(19)

Europäisches
Patentamt
European
Patent Office
Office européen
des brevets

(11) **EP 2 148 198 A1**

(12) **EUROPEAN PATENT APPLICATION**

(43) Date of publication:
**27.01.2010 Bulletin 2010/04**

(51) Int Cl.:
**G01N 33/44** *(2006.01)* **G01N 15/02** *(2006.01)*
**G01N 15/06** *(2006.01)* **G01N 30/00** *(2006.01)*
**G01N 35/08** *(2006.01)*

(21) Application number: **08305409.8**

(22) Date of filing: **21.07.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Centre National de la Recherche Scientifique
(CNRS)
75116 Paris (FR)**

(72) Inventors:
• **Cottet, Hervé
34000 Montpellier (FR)**

• **Matmour, Rachid
34070 Montpellier (FR)**
• **Biron, Jean Philippe
34980 Saint Gely du Fesc (FR)**
• **Martin, Michel
91220 Le Plessis-Pâté (FR)**

(74) Representative: **Domenego, Bertrand
Cabinet Lavoix
2, place d'Estienne d'Orves
75441 Paris Cedex 09 (FR)**

(54) **Determination of the hydrodynamic radii and/or content of constituents of a mixture by analysis of the Taylor dispersion of the mixture in a capillary tube**

(57) A method for analysing a mixture M comprising (i) a first monodisperse species, and (ii) a second species having a response coefficient which is distinct from the response coefficient of the first species (i) on at least one detection device, said method comprising the following steps:

(A) the mixture M is injected at the inlet of a capillary tube and forced to be transported in said tube by the flow of a carrier liquid induced by a positive hydrodynamic and/or hydrostatic pressure between the inlet and the outlet of the capillary, whereby a phenomenon of Taylor dispersion of the species of the mixture M occurs in the tube;
(B) by using a detection device able to detect simultaneously both species (i) and (ii) and placed in the region of the outlet of the capillary tube, a signal reflecting the Taylor dispersion obtained in step (A) is measured;
(C) the signal obtained in step (B) is analysed, so as to determine specific contributions of species (i) and (ii) and thereby establishing at least one of the followings:
- the content of species (i) and/or (ii) in the mixture M; and/or,
- the mean hydrodynamic radius of the species (ii) or (i).

FIG.1

EP 2 148 198 A1

**Description**

The instant invention relates to a method for analysing specific complex mixtures, which takes profit of the phenomenon of Taylor dispersion which takes place when species are mobilized in a capillary tube under hydrodynamic flow. The method of the invention, which allows to easily establish both the dimensions and the contents of the constituents of the mixture, is especially suitable for analysing a polymerization medium comprising a mixture including monomers and corresponding polymers.

In the field of analysis, a recurrent problem is the study of complex mixtures. Analysis of such mixtures often implies to separate the different constituents of the mixture (by the way of a chromatography, for example) before analysing the so separated constituents. The separation of the constituents may reveal to be difficult (and in certain cases almost impossible) to be carried out. Besides, such a separation is time consuming and further tends to affect the efficiency of the analysis.

Hence, analysis methods allowing obtaining direct information about the individual constituents of a mixture are of great interest. There are however very few analysis methods allowing such information to be obtained.

Especially, there is a need for a method which would allow obtaining information about the content of polymerization mixtures resulting from the polymerization of monomers or mixtures of polymers, especially about the remaining content of monomers (which reflects the degree of conversion) and the size of the formed polymers.

The instant invention aims at providing a new analysis method which allows to obtain direct information about the content and the size of the constituent of a mixture without having to preliminarily separate the constituents of the mixture.

In this connection, the invention especially aims at providing a method suitable for directly and simply analysing a polymerization medium including monomers and corresponding polymers and allowing to easily determine both the remaining content of monomers in the medium and the size of the formed polymers.

To this end, the instant invention provides a new method of analysis which makes use of the phenomenon of Taylor dispersion in a capillary tube. This new method is suitable for analysing specific mixtures, namely of the type including at least two kind of species which may be distinguished by a distinct response on a detection device.

More precisely, the invention relates to a method for analysing a mixture M comprising:

(i) a first species which is monodisperse; and,
(ii) a second species having a response coefficient which is distinct from the response coefficient of the first species (i) on at least one detection device, said method comprising the following steps:

(A) the mixture M is injected at the inlet of a capillary tube and forced to be transported in said tube by the flow of a carrier liquid induced by a positive hydrodynamic and/or hydrostatic pressure between the inlet and the outlet of the capillary tube, whereby a phenomenon of Taylor dispersion of the species of the mixture M occurs in the tube;
(B) by using a detector able to detect simultaneously both species (i) and (ii) and placed in the region of the outlet of the capillary tube, a signal reflecting the Taylor dispersion obtained in step (A) is measured;
(C) the signal obtained in step (B) is analysed by using an analysis device, so as to determine specific contributions of species (i) and (ii) and thereby establishing at least one of the followings:

-    the content of species (i) and/or (ii) in the mixture M ; and/or
-    the mean hydrodynamic radius of the species (ii) or species (i).

The method of the invention implements a Taylor dispersion of the species (i) and (ii) of the mixture M inside a capillary tube (step (A)) and a measurement of a signal reflecting the Taylor dispersion obtained in that way (step (B)), these steps being specifically followed by an analysis of the obtained signal (step (C)).

In practice, the signal of step (B) is generally obtained in the form of a diagram reflecting the evolution in time of the intensity detected by the detector placed in the region of the outlet of the capillary tube. This diagram, which is analogous to the chromatograms obtained with chromatographic methods, will be referred hereinafter as a "taylorgram", since it reflects the Taylor dispersion.

The Taylor dispersion which is used in the method of the invention is a well known phenomenon, which occurs when a species (especially in solution or dispersion) is forced to move in a hollow tube having a small internal diameter, by inducing a hydrodynamic flow in the tube. The hydrodynamic flow brings about a dispersive velocity profile within the tube, generally parabolic (of the Poiseuille profile type), the molecules or particles of a species which are closest to the wall of the tube having a displacement velocity which is almost zero, this velocity increasing as these molecules or particles move closer to the axis, with a maximum velocity for those which are located at the centre of the tube. It results a dispersive profile of the species at the outlet of the tube, reflected by a broadened peak on a taylorgram measured at the outlet of the tube. In this connection, reference may especially be made to the articles of G. Taylor, in Proc. Roy. Soc., A, 219, 186-203 (1953) and of R. Aris, in Proc. Roy. Soc. Lond. A., 235, 67-77 (1956)

It is well known from the prior art to make use of the Taylor dispersion for establishing the hydrodynamic radius of a single species. As a matter of fact, the hydrodynamic radius of a given species is related to the diffusion coefficient of this species, and said diffusion induces a more or less widening of the peak of the taylorgram (the lower the diffusion coefficient, the higher the broadening).

The analysis of the broadening for a single species (referred to as "Taylor Dispersion Analysis") allows direct establishment of hydrodynamic radius $R_h$ by using the following relationship:

$$H = \frac{kT}{3\pi\eta R_h u} + \frac{\pi\eta R_h d_c^2 u}{16kT}$$

Wherein:

- u is the linear displacement velocity of the species which is subject to the hydrodynamic flow of the carrier liquid;
- $d_c$ is the inner diameter of the tube used;
- T the absolute temperature;
- k is the Boltzmann constant
- $\eta$ is the viscosity of the carrier liquid in which the species is dispersed ; and,
- H is the plate height of the species (directly linked to the width of the detected peak), calculated as follows:

$$H = \frac{l_s \sigma_t^2}{t_d^2}$$

wherein:

- $l_s$ is the length travelled by the solute in the tube up to the detector,
- $t_d$ is the mean detection time of the peak;
- $\sigma_t^2$ is the time variance of the detected peak.

This relationship being written, for the specific example of a Gaussian peak:

$$H = \frac{l_s \delta^2}{5{,}54 . t_d^2}$$

where $\delta$ is the width of the peak at half-height.

As known in classical Taylor experiments, this expression of *H* can be modified to take into account some corrections due to the finite length of the injection plug and the pressure ramp. These corrections are described in the article by H. Cottet, M. Martin, A. Papillaud, E. Souaïd, H. Collet, A. Commeyras, Biomacromolecules, 2007, 8, 3235-3243. The average hydrodynamic radius of the solutes can be then derived from one taylorgram obtained at a given mobilization velocity u using the corrected *H* value according to the following equation:

$$R_h = \frac{2kT}{3\pi\eta u} \frac{1}{H - \sqrt{H^2 - \frac{d_c^2}{12}}}$$

Diffusion coefficient D and the hydrodynamic radius $R_h$ can be also obtained from the slope of the linear part of the H = f(u) curve, this slope being equal to: $\pi\eta R_h d_c^2 /(16kT)$ .

Another technique to get rid of this corrections in a certain extent, is to use a detection device having two points of measurements, whether two detectors along the capillary tube or a loop in the capillary tube effecting the mixture to pass twice in front of the same detector, $l_s$ being then the distance between the two points of detection along the capillary tube. This was described in the article by A. J. S. Chapman and D. M. Goodhall, Chromatography Today, Vol.1, June 2008, 22-24.

The Taylor Dispersion Analysis described hereinabove is not suitable for analysing media comprising a mixture of species. In fact, a direct Taylor dispersion analysis of a mixture conducted as described above leads to a global signal which reflects the global properties of the mixture, namely the average size of the whole species present in the mixture. This global information does not provide any indication on the specific properties of each of the species of the mixture, especially when the mixture contains monomers and corresponding polymers.

Unexpectedly, in the scope of the instant invention, the inventors have now found that taylorgrams which are obtained for most of the mixtures can actually be interpreted so as to establish respective contributions of each species which are part of the mixture. More precisely, the inventors have surprisingly evidenced that a deconvolution of the taylorgram of a mixture is possible when the mixture contains a first species of predetermined nature, known as having a monodisperse distribution (defined molecule or defined macromolecule, for example) and a second species having a response coefficient different from the response coefficient of the first species on a detection device.

As intended in the instant description, the expression "response coefficient" of a species on a given detection device denotes the proportionality coefficient between the detector signal and the concentration of the species, this concentration being appropriately defined.

More generally, the term "detection device" herein refers to any detection device which is useful for detecting the species (i) and (ii) at the end of the capillary tube of step (A), wherein the Taylor dispersion occurs, for example a UV detection device, an refractive index detection device, a light scattering detection device, a fluorescence detection device, or a conductivity detection device (for example of the type referred to as a "contactless conductivity detector"), a viscosity detection device, a mass spectrometer, an infrared detector, a NMR detector, an evaporative light scattering detector and the like.

Thus, the mean hydrodynamic radius of the species (i) or (ii) is a weight mean when using a mass concentration sensitive detector, a number mean when using a molar concentration sensitive detector or a z-mean when using a light scattering detector.

In fact, a great number of mixtures may be analysed according to the process of the invention, provided that they contain species which are sufficiently different in nature (as a matter of fact, a difference in nature generally implies that it exists at least one detector for which the response coefficients of the species will be distinct). Besides, a mixture analysed according to the process of the invention includes a monodisperse species (i).

The term "monodisperse species", as used herein, denotes a non-polymerized species (i.e. a defined molecule) or a population of polymerized macromolecules species wherein all the macromolecules have the same size. Especially, the method of the invention is suitable for analysing mixtures including (i) non-polymerized molecules of a predetermined nature, and (ii) macromolecules and/or aggregates and/or particles, for example mixtures including monomers and corresponding polymers.

The method of the invention may advantageously be carried out for analysing a mixture M which is a polymerization medium, and wherein species (i) is a monomer or a mixture of monomers and species (ii) is a polymer obtained by polymerization of said species. In that specific case, the followings are determined in step (C):

- the quantity of monomer (i) in the mixture M, which indicates the degree of conversion of the polymerization; and/or
- the mean hydrodynamic radius of the polymer (ii) (that allows monitoring the size of the formed polymer. If the Mark-Houwink equation is known for the formed polymer, the weight average molar mass $M_W$ of the formed polymer may further be deduced.)

The method of the invention may further be used for analysing other types of mixtures, such as, for example:

- mixtures including (i) proteins ; and (ii) protein aggregates or polymers;
- mixtures including (i) surfactants and monomers ; and (ii) latexes or polymers;
- mixtures including (i) nanoparticles ; and (ii) polymers.

The possibility of deconvolution of taylorgrams of mixtures, which has now been evidenced by the inventors, permits to directly obtain information about the mean hydrodynamic radii and the content of each of the species of the mixture, without having to preliminarily separate the constituents of said mixture. The process of the invention allows a one step very simple and fast analysis.

Moreover, the process of the invention may be carried out efficiently with extremely little quantities of the mixture to be analysed (quantities as small as 1 to 10 nL are generally injected). Furthermore, the process of the invention does not necessitate any expensive or bulky equipment.

Besides, steps (A) and (B) of the method of the invention may be implemented in almost all known capillary electrophoresis devices with no significant technical modification of these devices, which allows implementation of these steps (A) and (B) to be envisaged without any additional cost in most of existing commercial electrophoresis devices.

Especially due to these advantages, the method of the invention may be advantageously implemented both in the field of the search and on an industrial scale, especially for monitoring the evolution of reactions such as polymerization or for studying the stability of polymer / proteins formulations.

In the process of the invention, the analysis of the taylorgram (i.e. of the signal obtained in step (B)) is performed in step (C). In this step (C), the specific contributions of species (i) and (ii) on the signal obtained in step (B) are established, which allows to determine the content and/or the mean hydrodynamic radius of species (i) and/or (ii).

According to a first embodiment of the invention, the first species (i) being known to be monodisperse, the response coefficient of the species (i) and (ii) being known and the detection device implemented in step (B) being a device on which the species (i) an (ii) have two distinct response coefficients, step (C) is carried out as follows:

- performing an initial Taylor Dispersion experiment on species (i) only to derive the value of the area $A_0$, the height $h_0$ and standard deviation $\sigma_i$ from an initial signal $S_0(t)$ ; and,
- on the global signal $S_n(t)$ provided at the step (B) on sample n, calculating:

$$\alpha_n = \frac{h_n}{h_0}\frac{A_0}{A_n} \qquad \text{and} \qquad \beta_n = \frac{\sigma_n^2}{\sigma_i^2}$$

where $A_n$, $h_n$ and $\sigma_n^2$ are respectively the area, the peak height and the variance of the global signal;

- calculating the degree of conversion of the polymerization

$$\Psi_n = \frac{y_n}{y_n + \kappa(1 - y_n)},$$

through the determination of:

$$x_n = \frac{\sigma_{ii}}{\sigma_i} = \frac{1}{2}\left(\sqrt{\frac{4\beta_n - 3 - \alpha_n}{1 - \alpha_n}} - 1\right) \text{ and } y_n = \frac{A_{ii}}{A_n} = (\beta_n - \alpha_n)\frac{x_n}{x_n^3 - 1} \ .$$

When two species (i) and (ii) have distinct hydrodynamic radii, they generally have distinct response coefficients on a light scattering detection device. Thus, with such species, the method according to the first embodiment of the invention may be carried out by implementing a light scattering detection device.

However, with such species having distinct hydrodynamic radii (especially monomers and corresponding polymers; or proteins or proteins aggregates), another method may be employed, which takes profit of this difference of hydrodynamic radii. In this case, the difference of hydrodynamic radii offers the possibility of directly establishing the contributions of species (i) in the signal obtained in step (B) by a deconvolution of the signal.

More precisely, according to a second embodiment of the invention, which is suitable when the species (i) an (ii) have two distinct hydrodynamic radii and when species (i) is a species of a predetermined nature, the method is carried out such that, in step (C), the respective contributions of species (i) and (ii) in the signal obtained in step (B) are established, whereby the elution profiles of species (i) and (ii) are obtained, which allows to determine the content of species (i) and/or (ii) in the mixture M, and/or the mean hydrodynamic radius of the species (i) and/or (ii).

This second embodiment of the method of the invention is especially advantageous since it does not need any initial Taylor experiment.

The method according to the second embodiment of the invention is especially suitable for analysing mixtures M of the type including:

(i) non-polymerized molecules of a predetermined nature, and
(ii) macromolecules and/or aggregates and/or particles.

With such mixtures, the method according to the second embodiment of the invention allows to establish in step (C):

- the content of species (i) and/or (ii) in the mixture M ; and/or
- the mean hydrodynamic radius of species (ii).

More generally, the method according to the second embodiment of the invention is suitable for analyzing any mixture containing (i) monodisperse species of a predetermined nature (such as proteins for example); and (ii) aggregates and/or particles (proteins aggregates for example).

According to a first alternative of the method of the second embodiment of the invention, considering that the elution profile of species (i) is a Gaussian distribution, the respective contributions of species (i) and (ii) in the signal obtained in step (B) are established in step (C) by:

- fitting a first Gaussian distribution onto the global signal $S_n(t)$, resulting in a first fitted Gaussian distribution corresponding to the elution profiles of species (i);
- subtracting from the signal the first fitted Gaussian distribution, resulting in a reduced signal $S'_n(t)$ corresponding to the elution profile of species (ii) and providing information about species (ii).

In this first alternative, preferably, the reduced signal $S'_n(t)$ is processed to obtain a value of the variance for species (ii) according to:

$$\sigma_{ii,n}^2 = \frac{\int_{t_{d,n}-b}^{t_{d,n}+b} S_n'(t).(t-t_{d,n})^2.dt}{\int_{t_{d,n}-b}^{t_{d,n}+b} S_n'(t).dt} \text{ or } \sigma_{ii,n}^2 = \frac{\int_{t_{d,n}-b}^{t_{d,n}} S_n'(t).(t-t_{d,n})^2.dt}{\int_{t_{d,n}-b}^{t_{d,n}} S_n'(t).dt}$$

where b is a period of time large enough for the signal to vanish at $t_{d,n} - b$ and at $t_{d,n} + b$.

In a second possible alternative of the second embodiment of the invention, considering that the elution profile of species (i) and species (ii) are Gaussian distributions, the respective contributions of species (i) and (ii) in the signal obtained in step (B) are established, in step (C), by fitting a function which is the sum of first and second Gaussian distributions onto the signal $S_n(t)$, resulting in first and second fitted Gaussian distributions corresponding to the elution profiles of species (i) and (ii).

Then, the value of the variance of the elution profile of species (ii) is used to determine, a hydrodynamic radius according to the equation:

$$R_{h,ii,n} = \frac{2kT}{3\pi\eta u} \frac{1}{H_{ii,n} - \sqrt{H_{ii,n}^2 - \frac{d_c^2}{12}}}$$

with:

$$H_{ii,n} = \frac{l_s \sigma_{ii,n}^2}{t_d^2}$$

Preferably, the value of the area of the elution profiles of species (i) and (ii) are used to determine, the degree of conversion $\Psi_n$ according to the equation:

$$\Psi_n = \frac{A_{m,0} - A_{m,n}}{A_{m,0}}$$

where $A_{m,0}$ and $A_{m,n}$ are the areas of the monomer signals at time 0, before the start of the polymerization and in experiment n during the polymerization process, respectively.

The mixture M being a polymerization medium, and the species (i) being a monomer and the species (ii) being polymers obtained by polymerization of said species (i), the followings are determined in step (C) :

- the quantity of monomer (i) in the mixture M, which indicates the degree of conversion of the polymerization; and/or
- the mean hydrodynamic diameter of the polymer (ii).

Preferably, the mixture M is diluted or dissolved in a medium identical to the carrier liquid used in the Taylor dispersion of step (A), before injecting it in the capillary.

The internal surface of the capillary tube is non-covalently or covalently coated with a compound which limits or inhibits the interaction between the species (i) and (ii) and the inner surface of the capillary tube, said coated compound being chosen from the group consisting in PEO, cellulose derivatives, polyvinyl alcohol, polyacrylamides, polysiloxanes such as polydimethylsiloxane, anionic or cationic (mono- or double-chain) surfactants.

The mixture M is introduced in the capillary tube together with a carrier liquid (a good solvent of the solute mixture). When using water or hydro-organic solvents, the carrier liquid generally includes a salt, for example NaCl, or a buffered solution.

The method, wherein a plurality of samples, which each include a mixture of at least two species and having two different response coefficients on at least one detection device, are analysed according to steps (A) to (C) as defined here above in the same capillary tube, in a sequential way, the samples being injected successively in said capillary tube.

The object of the invention is also an analysis device comprising memorisation means, a processing means and an I/O interface to which is connected a detector capable of providing a Taylor signal, the analysis device comprising means for the implementation of the analysis method which has just been introduced.

Different variants and preferred embodiments of the method of the invention will now be described in greater details with reference to the attached drawings, on which:

- Figure 1 is a schematical block illustration of a device capable of analysing in real time a taylorgram; and,
- Figure 2 is an algorithmic representation of the method of treatment performed by the device of figure 1.

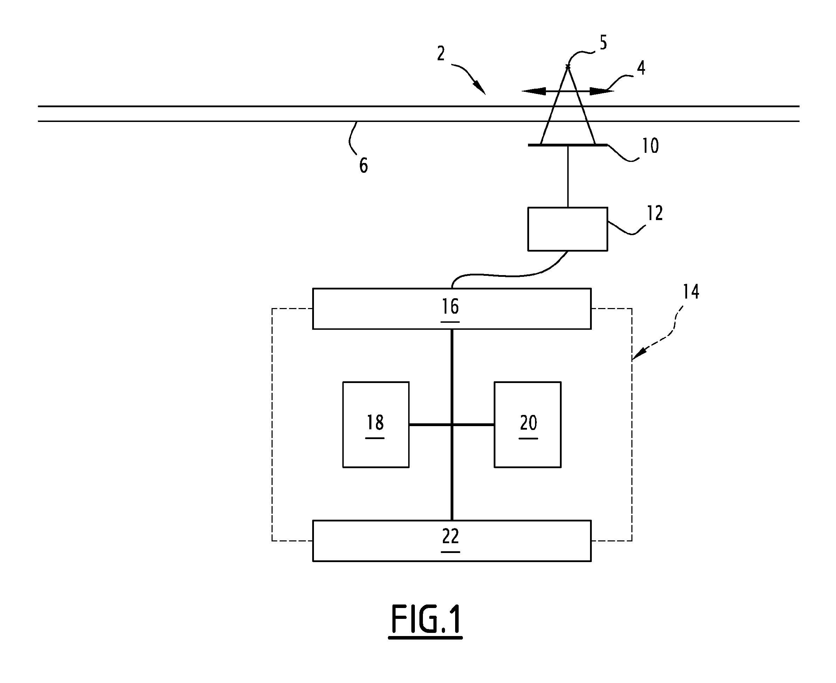

As illustrated in figure 1, a detector 2 for a Taylor experiment contains a light source S and an optical system 4 to irradiate capillary tube 6 through which flows the mixture to be analysed. Detector 2 also contains an optical sensor 10 coupled to an electronic board 12 capable of generating an electrical signal S. The level of signal S depends directly on the amount of light incident on sensor 10 during a period of time equal to the sampling time.

Detector 2 is connected to an analysis device 14 through an Input/Output interface 16. Device 14 further comprises memorisation means, such as RAM and/or ROM 18, and processing means, such that CPU 20. Device 14 is also provided with means to allow an operator to interact with software running on device 14. For example, device 14 is equiped with a tactile screen 22.

The method of treatment of the electrical signal S that will be described hereafter in details is preferably performed by a software comprising instructions memorised in ROM 18 and processed by CPU 20.

In step (A), the mixture M, as such or dissolved and/or dispersed in a proper solvent or dispersant, is injected in the capillary tube and forced to be transported in this capillary tube by the flow of a carrier liquid induced by a positive hydrodynamic and/or hydrostatic pressure difference between the inlet and the outlet of the capillary.

The capillary tube 6 used in step (A) has advantageously an inner diameter of between 5 and 500 $\mu$m. The inner diameter of the capillary is advantageously less than or equal to 300 $\mu$m, preferably less than or equal to 100 $\mu$m,

especially in order to prevent an excessively high level of dispersion of the peaks. However, it is preferable for this inner diameter to remain greater than or equal to 10 $\mu$m, in particular in order to allow adequate sensitivity of measurement and also to provide for conditions under which the plate height $H$ is a linear function of the mobilizing velocity u. Thus, typically, the inner diameter of the capillary tube used in step (A) is between 25 and 100 $\mu$m. Thus, as capillaries which can advantageously be used to implement step (A), mention may be made to conventional capillaries having an inner diameter of 10 $\mu$m, 25 $\mu$m, 50 $\mu$m, 75 $\mu$m or 100 $\mu$m, capillaries of 25, 50 and 75 $\mu$m being found to be particularly suitable in most cases.

As it is well known in the field of the Taylor dispersion, the length $l$ of the capillary tube implemented in the method of the invention has to be sufficiently high so as to obtain a detection time well greater than the characteristic diffusion time (which is calculated by the ratio $R^2/4D$ wherein R is the internal radius of the capillary tube 6 and $D$ is the diffusion coefficient). To this end, the length of the capillary tube used in the method of the invention is advantageously of at least 10 cm, preferably of at least 20 cm. Besides, especially in order to limit the analysis times, it is preferable for the length of the capillary to remain less than 100 cm, for example, less than or equal to 50 cm. In the meaning of the instant description, the term "length of a capillary tube" intends to denote the effective length of the tube, namely the length from the inlet to the optical sensor 10 located in the region of the outlet of the capillary tube.

The Taylor dispersion which is conducted in step (A) may be carried out in accordance with any method known per se, for example, in accordance with the technique described in J. Phys. Chem., 1974, 78, 2297-2301 or in Science, 1994, 266, 773-776.

In the method of the invention, the Taylor dispersion is brought about by establishing in step (A) a positive hydrodynamic and/or hydrostatic pressure difference between the inlet and the outlet of the capillary tube.

In step (A) of the method of the invention, it is highly preferable that the linear displacement velocity u of the species (i) and (ii) in the capillary tube fulfils the following relation:

$$7\frac{D}{R} < u << \frac{4Dl}{R^2}$$

It is well known by the skilled person to adapt the hydrodynamic and/or hydrostatic pressure difference induced between the inlet and the outlet of the capillary tube so as to attain the sought rate of flow for a given capillary. For example, in the case of species having diffusion coefficients of about $10^{-9}$ to $10^{-11}$ m$^2$s$^{-1}$, and for a capillary tube having an internal diameter of 50 $\mu$m and an efficient length of 30 cm, the mobilisation rate has to be between $5.10^{-4}$ and $2.10^{-3}$ ms$^{-1}$, which corresponds to a pressure difference between the inlet and the outlet of the capillary tube of about 10 to 50 mbar ($10^3$ to $5.10^3$ Pa). For species having diffusion coefficients of less than $10^{-11}$ m$^2$s$^{-1}$, it often reveals suitable to make use of capillary tube having an internal diameter of less than 50 $\mu$m so as to obtain reasonable mobilisation rates and analysis times. More generally, the hydrodynamic and/or hydrostatic pressure difference between the inlet and the outlet of the capillary in step (A) may be from 1 to 500 mbar (100 Pa to 50 000 Pa) especially for capillary tube having an internal diameter of 25 to 100 $\mu$m. Greater pressure difference will be more suitable for capillary tube having an internal diameter of less than 25 $\mu$m.

Furthermore, it is generally preferable for the pressure difference applied during step (A) between the ends of the capillary to remain substantially constant for the entire duration of step (A), in particular in order to allow the most precise measurement possible of the hydrodynamic radius in step (C). In this manner, advantageously, during step (C), the pressure varies at the most within +/- 0.5 mbar (50 Pa) of a fixed reference value. However, the value of this reference value most generally does not have to be determined in a precise manner.

In step (A), the pressure difference between the two ends of the capillary may be established in accordance with any method known per se, for example, by applying excess pressure in the region of the inlet of the capillary or, conversely, by applying reduced pressure at the outlet. According to a more specific embodiment, the pressure difference between the two ends of the capillary may be brought about by establishing a level difference between reservoirs of solvent or mobile phase at the inlet and at the outlet of the capillary. This embodiment is generally found to be advantageous since it allows a constant pressure difference to be established for the entire duration of step (A) without requiring any additional pressure regulation system.

In step (A), it is generally preferable to dilute or dissolve the mixture M in a medium identical to the carrier liquid used in the Taylor experiment before injecting it in the capillary tube 6. This pre-dilution or pre-solubilisation of the mixture M especially allows:

- a limitation or an inhibition of baseline perturbations of the signal obtained in step (B) which may otherwise occur (especially when the detection device located in the region of the outlet of the capillary tube is a UV-detector: in the absence of a dilution or solubilisation of the mixture, modifications of the refraction index of the mixture may occur in the injection zone). This "stabilisation" of the signal obtained in step (B) renders more efficient the analysis of step (C).

- a limitation or an inhibition of modification of the conformation of macromolecules (polymers or proteins for example) present in the mixtures during the Taylor dispersion experiment. The dilution or solubilisation tends to level off the change in ionic strength between the mixture zone and the carrier liquid.
- a lower change in the viscosity between the mixture zone and the mobile phase.
- a limitation of intermolecular interactions between sample (macro)molecules that are assumed to be negligible for calculation of the radius $R_h$.

Besides, in step (A), the internal surface of the capillary is preferably non-covalently or covalently coated with a compound which limits or inhibits the interaction (especially adsorption) between the species (i) and (ii) and the inner surface of the capillary, said coated compound being preferably chosen from the group consisting in PEO, cellulose derivatives, polyvinyl alcohol, polyacrylamides, polysiloxanes such as polydimethylsiloxane, or surfactants (mono-chain or double chain surfactants). Polyelectrolyte multilayer coatings may be applied on the internal face of the capillary tube. Commercial coatings such as DB1, DB17 or DB225 are especially useful in this connection.

The inhibition of the interactions between the capillary and the species (i) and (ii) improves the quality of the signal obtained in step (B) and hence facilitates the analysis of this signal in step (C).

Any other means allowing to limit or inhibit interactions between species (i) and (ii) may be advantageously implemented in the step (A). Especially, according to a specific embodiment, it may reveals advantageous that the mixture M is introduced in the capillary tube together with a carrier liquid including a salt, for example NaCl.

According to an advantageous alternative, the method of the invention may be conducted so as to sequentially analyse a plurality of N samples. In this alternative, a plurality of samples, which each includes a mixture of at least two species and having two different response coefficients on at least one detection device, are analysed, according to steps (A) to (C) as defined in claim 1, in the same capillary tube, in a continuous way, the samples being injected successively in said capillary tube. According to this embodiment, it is not necessary to wait the detection of a first sample detected and analysed according to step (B) and (C) before introducing the following sample. Thus, in this connection, the instant invention provides an efficient and fast analysis method, which allows a high flow of sample. This embodiment of the method of the invention is, inter alia, especially suitable for a continuous study of the evolution of a polymerization.

By using device 14 and detector 2 on the $n^{th}$ sample of mixture M of monomer m and polymer p, the signal $S_n$ (t) given by the sensor at the end of step (B) is the superposition of the signal $S_{m,n}(t)$ of the monomer m and the signal $S_{p,n}(t)$ of the polymer p.

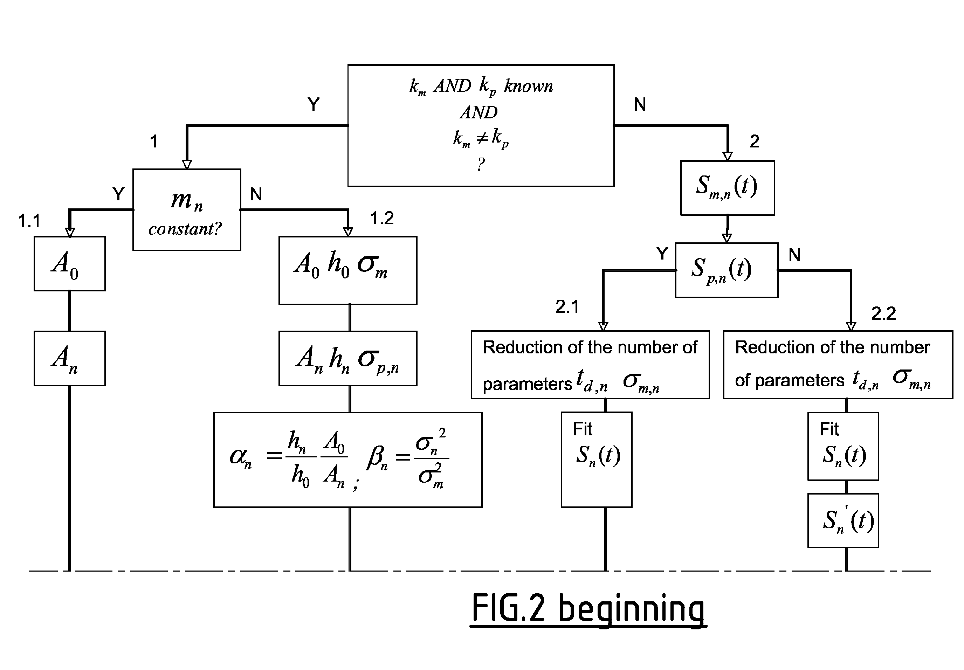

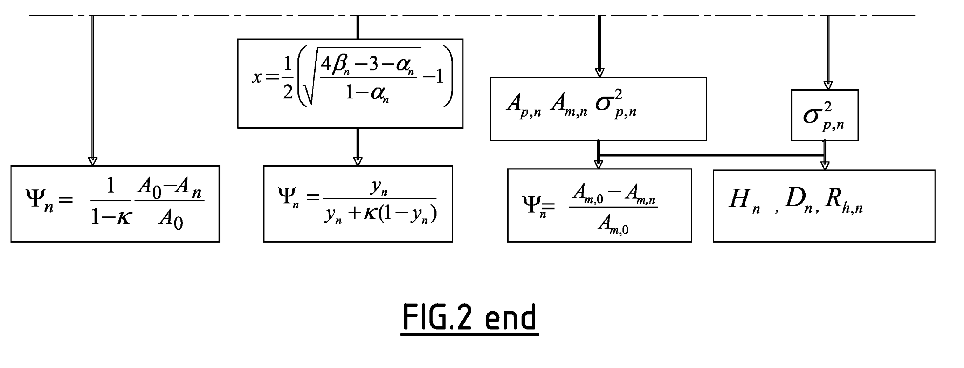

To derive further physical data on these two species, such that the degree of conversion of the polymerisation reaction and/or the mean hydrodynamic radius of the polymer p, each contribution to the global signal $S_n(t)$ must be separated by applying one of the following methods of treatment of the global signal $S_n(t)$, as it can be seen in figure 2.

For example, the degree of conversion $\Psi_n$ of the polymerisation reaction at the time sample n is taken off, is defined as:

$$\Psi_n = \frac{m_{p,n}}{m_{m,n} + m_{p,n}} \tag{1}$$

where $m_{m,n}$ and $m_{p,n}$ are the masses, respectively of the monomer m and the polymer p, in sample n.

<u>1. The response coefficients of each species are known</u>

The response coefficient k for one species is defined by:

$$A_{m,n} = k_m . m_{m,n} \tag{2}$$

$$A_{p,n} = k_p.m_{p,n} \qquad (3)$$

where $A_{m,n}$ and $A_{p,n}$ are the areas of the corresponding signals $S_{m,n}(t)$ and $S_{p,n}(t)$.

Coefficients $k_m$ and $k_p$ are considered constant throughout the experiment, even if the structure of a species, like the one of the polymer p, can evolve.

1.1. With a condition of conservation of the mass

The mass of sample n can be expresses as the sum of the mass of the monomer and the mass of the polymer:

$$m_n = m_{m,n} + m_{p,n} \qquad (4)$$

where $m_n$ can be considered as the mass introduced in the device for experiment n.

One hypothesis is to consider $m_n$ constant, and equal to $m$, from one sample to the other.

The total area $A_n$ of total signal $S_n(t)$ is:

$$A_n = k_m m_{m,n} + k_p m_{p,n} \qquad (5)$$

At the beginning (n=0) of the polymerisation, the sample only contains a mass $m$ of monomer, so:

$$A_0 = k_m.m_{m,0} = k_m.m \qquad (6)$$

Now, equation (1) can be written as:

$$\Psi_n = \frac{1}{1-\kappa} \frac{A_0 - A_n}{A_0} \qquad (7)$$

with:

$$\kappa = \frac{k_p}{k_m} \qquad (8)$$

Thus, the degree of conversion $\Psi_n$ can be directly calculated from the total area $A_0$ and $A_n$ of the signal before polymerisation (n=0) and at the current time n.

This is possible when the mass of the sample is constant and if $k_p$ is different from $k_m$ in order the denominator of equation (7) does not vanish.

1.2. Without a condition of conservation of the mass

When considering that the condition about the mass of the sample which must be constant throughout several samples is too restrictive or difficult to put into practice, the following method can be used.

Assuming that the mean time $t_{d,m,n}$ and $t_{d,p,n}$ of the two signals $S_{m,n}(t)$ and $S_{p,n}(t)$ are the same, and that the two signals $S_{m,n}(t)$ and $S_{p,n}(t)$ are symmetrical around this common mean time $t_{d,n}$, then the maximum $h_n$ of the global signal $S_n(t)$ arises at $t_{d,n}$ and is given by:

$$h_n = h_{m,n} + h_{p,n} \qquad (9)$$

where $h_{m,n}$ and $h_{p,n}$ are the heights of the peaks of the signals $S_{m,n}(t)$ and $S_{p,n}(t)$, respectively.

Similarly, the variance $\sigma_n^2$ of the global signal $S_n(t)$ can be expressed, by:

$$\sigma_n^2 = \frac{A_{p,n}}{A_{m,n} + A_{p,n}} \sigma_{p,n}^2 + \frac{A_{m,n}}{A_{m,n} + A_{p,n}} \sigma_{m,n}^2 \qquad (10)$$

where $\sigma_{m,n}^2$ and $\sigma_{p,n}^2$ are the variances of the $S_{m,n}(t)$ and $S_{p,n}(t)$ signals, respectively.

By introducing parameter $y_n$ defined by:

$$y_n = \frac{A_{p,n}}{A_{m,n} + A_{p,n}} \qquad (11)$$

it is easy to derive that:

$$y_n = \frac{\kappa \, \Psi_n}{1 - \Psi_n + \kappa \Psi_n} \qquad (12)$$

or, that:

$$\Psi_n = \frac{y_n}{y_n + \kappa(1 - y_n)} \qquad (13)$$

The two parameters of interest are $\sigma_p^2$ and $\Psi_n$. The latter is connected to the areas $A_{m,n}$ and $A_{p,n}$ via equations (11) and (13).

The method involves the introduction of two new variables, the values of which are accessible from the experiment:

$$\alpha_n = \frac{h_n}{h_0} \frac{A_0}{A_n} \qquad (14)$$

$$\beta_n = \frac{{\sigma_n}^2}{{\sigma_m}^2} \qquad (15)$$

It can be shown that:

$$\alpha_n = 1 - y_n + \frac{y_n}{x} \qquad (16)$$

$$\beta_n = 1 - y_n + y_n x^2 \qquad (17)$$

Where parameter $x_n$ is defined by:

$$x_n = \frac{\sigma_{p,n}}{\sigma_{m,n}} \qquad (18)$$

Equations (16) and (17) lead to:

$$x_n = \frac{1}{2}\left( \sqrt{\frac{4\beta_n - 3 - \alpha_n}{1 - \alpha_n}} - 1 \right) \qquad (19)$$

$$y_n = (\beta_n - \alpha_n)\frac{x_n}{x_n^3 - 1} \qquad (20)$$

and, the value $\Psi_n$ can be derived from $y_n$ using equation (13) and $\sigma_p$ using equation (18).
Thus, according to this method, the measurements of the area $A_0$, the height $h_0$ and the standard deviation $\sigma_m$ of the signal $S_{m,0}(t)$ of the monomer before the polymerisation reaction, allow the determination of the degree of conversion $\Psi_n$ and of the standard deviation $\sigma_p$ of the polymer.

### 2. Hypothesis on the response signals

Considering that the signal $S_{m,n}(t)$ of the monomer is a Gaussian distribution, we have:

$$S_{m,n}(t) = \frac{A_{m,n}}{\sigma_{m,n}\sqrt{2.\pi}}.\exp\left\{ -\frac{1}{2}\left[ \frac{t - t_{d,m}}{\sigma_m} \right]^2 \right\} \qquad (21)$$

where $\sigma_m$ is the standard deviation, $t_{d,m}$ the mean value and $A_{m,n}$ a normalisation factor corresponding to the area of the signal $S_{m,n}(t)$.

2.1.

The signal of the polymer $S_{p,n}(t)$ can also be approximated with a Gaussian distribution:

$$S_{p,n}(t) = \frac{A_{p,n}}{\sigma_{p,n}\sqrt{2.\pi}}.\exp\left\{-\frac{1}{2}\left[\frac{t-t_{d,p}}{\sigma_{p,n}}\right]^2\right\} \qquad (22)$$

This is the case when the dispersion in molar mass of the polymer p is not too large.

Thus, a first method to separate each contribution consists in fitting a function which is the sum of two Gaussian distributions onto the global signal $S_n(t)$.

This fit involves six parameters, namely $\sigma_m$, $\sigma_{p,n}$, $t_{d,m}$, $t_{d,p}$, $A_{m,n}$ and $A_{p,n}$.

It is possible to reduce the number of parameters in order to get a better precision from the fit.

For example, $\sigma_m$ is a characteristic parameter of the monomer. It can be measured trough an initial Taylor experiment with a solution containing only the monomer.

It is to be noticed that because Taylor dispersion is not a method of separation of the compounds of the mixture, $t_{d,m}$ is egal to $t_{d,p}$, and is also equal to the mean value $t_{d,n}$ of the global signal $S_n(t)$.

2.2.

When the molar mass distribution of the polymer p is too polydispersed, it is no longer possible to fit a Gaussian distribution onto the global signal $S_n(t)$. In this case, the method consists in first fitting a Gaussian distribution onto the signal of the monomer $S_m(t)$, then subtracting the fitted Gaussian distribution from the signal $S_n(t)$ to obtain a reduced signal $S_n'(t)$ and then calculating the variance of the reduced signal $S_n'(t)$ as an approximation of $\sigma_{p,n}^2$ :

$$\sigma_{p,n}^2 = \frac{\int_{t_{d,n}-b}^{t_{d,n}+b} S_n'(t).(t-t_{d,n})^2.dt}{\int_{t_{d,n}-b}^{t_{d,n}+b} S_n'(t).dt} \qquad (23)$$

This method works well when the characteristic parameters of the signal of the monomer $S_{m,n}(t)$ are significantly different from those of the signal of the polymer $S_{p,n}(t)$, i.e. when the signal of the monomer $S_{m,n}(t)$ can be "visually" separated from the signal of the polymer $S_{p,n}(t)$. For example, when the signal of the monomer $S_{m,n}(t)$ is narrow and sharp whereas the signal of the polymer $S_{p,n}(t)$ is broad (i.e. when the polymer has much larger molar mases than the monomer).

The implementation of this method in device 14 can be done by fitting a Gaussian distribution onto the global signal $S_n(t)$ but only inside a reduced window centered on the maximum height of the global signal.

Alternatively, an iteration process can be used: in a loop of this iteration process, a fit corresponding to the monomer signal is adjusted on the global signal $S_n(t)$. The fitted Gaussian distribution is subtracted from the global signal $S_n(t)$ to get the reduced signal $S_n'(t)$. Then, the shape of the reduced signal $S_n'(t)$ is tested. This test may consist in calculating the number of zeros of the derivative of the reduced signal $S_n'(t)$: if the derivative has three zeros, it means that the area of the fitted Gaussian distribution is too large and that a part of the polymer signal has been considered as belonging to the monomer signal. Thus, in the following loop of the iteration process, the area of the Gaussian to be fitted is decreased. On the contrary, if the derivative has only one zero, it means that the area of the fitted Gaussian distribution is too small and that a part of the monomer signal has not been taken into account in the monomer signal. Thus, in the following loop of the iteration process, the area of the Gaussian to be fitted is increased. The convergence of such an iteration process results in a limit by excess of the area of the monomer signal. Another criterion can be used such that the number of zeros of the second derivative of the resulted signal $S_n'(t)$.

In experimental conditions, it may happen that the signal $S_{p,n}(t)$ of the polymer has a tail due to an absorption phenomenon of the polymer on the wall of the capillary tube. In order to avoid the calculation of $\sigma_{p,n}^2$ be biased by this phenomenon and considering the signal as symmetrical, equation (23) becomes:

$$\sigma_{p,n}^2 = \frac{\int_{t_{d,n}-b}^{t_{d,n}} S_n'(t).(t-t_{d,n})^2.dt}{\int_{t_{d,n}-b}^{t_{d,n}} S_n'(t).dt} \qquad (24)$$

It is to be noticed that $A_{m,n}$ can be derived from the result of the fitting of a Gaussian distribution and that $A_{p,n}$ can be obtained by subtracting $A_{m,n}$ from $A_n$. From the deconvolution of the respective contributions of the monomer and the polymer, it is possible to get the degree of conversion $\Psi_n$ for sample n:

$$\Psi_n = \frac{A_{m,0} - A_{m,n}}{A_{m,0}} \qquad (25)$$

These data on the elution profiles of the monomer and the polymer can be used to determine physical characteristics such that the hydrodynamic radius of the polymer. The radius $R_{h,p,n}$ is connected to the diffusion coefficient $D_{p,n}$ by means of the Stokes-Einstein relationship:

$$R_{h,p,n} = \frac{kT}{6\pi\eta D_{p,n}} \qquad (26)$$

and the diffusion coefficient $D_{p,n}$ is connected to the variance $\sigma_{p,n}^2$ through parameter $H_{p,n}$:

$$D_{p,n} = \frac{u}{4}\left( H_{p,n} - \sqrt{H_{p,n}^2 - \frac{d_c^2}{12}} \right) \qquad (27)$$

$$H_{p,n} = \frac{l_s.\sigma_{p,n}^2}{t_d^2} \qquad (28)$$

where $l_s$ is the length of the capillary tube from the injection point to the detector and $u$ is the flow velocity of the carrier solvent through the tube, $\eta$ is the viscosity of carrier liquid at temperature T. So, once $\sigma_{p,n}^2$ has been measured for sample n, a hydrodynamic radius $R_{h,p,n}$ can be derived for the polymer of this $n^{th}$ sample.

In the software implementation of this method, $D_{p,n}$ is only an intermediary parameter. It is not necessary to use it explicitly in the calculation of the value of the radius $R_{h,p,n}$ which can be derived directly from the input of $\sigma_{p,n}^2$:

$$R_{h,p,n} = \frac{2kT}{3\pi\eta u} \frac{1}{H_{p,n} - \sqrt{H_{p,n}^2 - \frac{d_c^2}{12}}} \quad \text{with} \quad H_{p,n} = \frac{l_s \sigma_{p,n}^2}{t_d^2} \quad\quad (29)$$

**Claims**

1. A method for analysing a mixture M comprising:

   (i) a first species which is monodisperse, and
   (ii) a second species having a response coefficient which is distinct from the response coefficient of the first species (i) on at least one detection device, said method comprising the following steps:

   (A) the mixture M is injected at the inlet of a capillary tube and forced to be transported in said tube by the flow of a carrier liquid induced by a positive hydrodynamic and/or hydrostatic pressure between the inlet and the outlet of the capillary tube, whereby a phenomenon of Taylor dispersion of the species of the mixture M occurs in the tube;
   (B) by using a detector able to detect simultaneously both species (i) and (ii) and placed in the region of the outlet of the capillary tube, a signal reflecting the Taylor dispersion obtained in step (A) is measured;
   (C) the signal obtained in step (B) is analysed by using an analysis device, so as to determine specific contributions of species (i) and (ii) and thereby establishing at least one of the followings:

   - the content of species (i) and/or (ii) in the mixture M ; and/or
   - the mean hydrodynamic radius of the species (ii) or species (i).

2. The method of claim 1 wherein the mean hydrodynamic radius of the species (ii) is a weight mean, a number mean or a z-mean.

3. The method of claims 1 or 2, wherein the first species (i) is known to be monodisperse, the response coefficient of the species (i) and (ii) are known and the detection device implemented in step (B) is a device on which the species (i) an (ii) have two distinct response coefficients, and wherein the signal analysis of step (C) is carried out and wherein the signal analysis of step (C) is carried out as follows:

   - performing an initial calibration experiment on species (i) only to derive the value of the area $A_0$ , the height $h_0$ and standard deviation $\sigma_i$ from an initial signal $S_0(t)$ ; and,
   - on the signal $S_n(t)$ provided at the step (B) on sample n, calculating:

   $$\alpha_n = \frac{h_n}{h_0} \frac{A_0}{A_n} \quad\quad \text{and} \quad\quad \beta_n = \frac{\sigma_n^2}{\sigma_i^2}$$

   where $A_n$ , $h_n$ and $\sigma_n^2$ are respectively the area, the peak height and the variance of the total signal;

   - calculating the degree of conversion of the polymerization

   $$\Psi_n = \frac{y_n}{y_n + \kappa(1 - y_n)},$$

   through the determination of:

$$x = \frac{1}{2}\left(\sqrt{\frac{4\beta_n - 3 - \alpha_n}{1 - \alpha_n}} - 1\right) \text{ and } y_n = \frac{A_{ii}}{A_n} = (\beta_n - \alpha_n)\frac{x}{x^3 - 1} \; .$$

4. The method of claims 1 or 2, wherein the species (i) an (ii) have two distinct hydrodynamic radii, and species (i) is a species of a predetermined nature and wherein, in step (C), the respective contributions of species(i) and (ii) in the signal obtained in step (B) are established, whereby the elution profiles of species (i) and (ii) are obtained, which allows to determine the content of species (i) and/or (ii) in the mixture M, and/or the mean hydrodynamic radius of the species (i) and/or (ii).

5. The method of claim 4, wherein the mixture M includes:

(i) non-polymerized molecules of a predetermined nature, and
(ii) macromolecules and/or aggregates and/or particles, and wherein at least one of the followings is determined in step (C):

- the content of species (i) and/or (ii) in the mixture M; and/or,
- the mean hydrodynamic radius of species (i) and/or (ii).

6. The method of claims 4 or 5, wherein considering that the elution profile of species (i) is a Gaussian distribution, the respective contributions of species (i) and (ii) in the signal obtained in step (B) are established in step (C) by:

- fitting a first Gaussian distribution onto the signal, resulting in a first fitted Gaussian distribution corresponding to the elution profiles of species (i);

- subtracting from the signal the first fitted Gaussian distribution, resulting in a reduced signal $S'_n(t)$ corresponding to the elution profiles of species (ii) and providing information about species (ii).

7. The method of claim 6, wherein the reduced signal $S'_n(t)$ is processed to obtain a value of the variance for species (ii) according to the equation:

$$\sigma^2_{ii,n} = \frac{\displaystyle\int_{t_{d,n}-b}^{t_{d,n}+b} S'_n(t).(t - t_{d,n})^2.dt}{\displaystyle\int_{t_{d,n}-b}^{t_{d,n}+b} S'_n(t).dt}$$

where b is a period of time large enough for the signal to vanish at $t_{d,n}$ - b and at $t_{d,n}$ + b.

8. The method of claim 6, wherein, considering that the reduced signal $S'_n(t)$ is not symmetrical, the resulted signal is processed to obtain a value of the variance for species (ii) according to the equation:

$$\sigma^2_{ii,n} = \frac{\displaystyle\int_{t_{d,n}-b}^{t_{d,n}} S'_n(t).(t - t_{d,n})^2.dt}{\displaystyle\int_{t_{d,n}-b}^{t_{d,n}} S'_n(t).dt} \; .$$

9. The method claim 1, wherein, considering that the elution profile of species (i) and species (ii) are Gaussian distributions, the respective contributions of species (i) and (ii) in the signal $S_n(t)$ obtained in step (B) are established, in step (C), by fitting a function which is the sum of first and second Gaussian distributions onto the signal $S_n(t)$, resulting in first and second fitted Gaussian distributions corresponding to the elution profiles of species (i) and (ii).

10. The method of anyone of claims 7 to 9, wherein the value of the variance of the elution profile of species (ii) is used to determine a hydrodynamic radius according to the equation:

$$R_{h,ii,n} = \frac{2kT}{3\pi\eta u} \frac{1}{H_{ii,n} - \sqrt{H_{ii,n}^2 - \frac{d_c^2}{12}}} \quad \text{with} \quad H_{ii,n} = \frac{l_s.\sigma_{ii,n}^2}{t_d^2}.$$

11. The method of anyone of claims 6 and 10, wherein the value of the area of the elution profiles of species (i) and (ii) are used to determine, the degree of conversion $\Psi_n$ according to the equation:

$$\Psi_n = \frac{A_{m,0} - A_{m,n}}{A_{m,0}}.$$

12. The method of anyone of claims 1 to 11, wherein the mixture M is a polymerization medium, and wherein the species (i) is a monomer and the species (ii) are polymers obtained by polymerization of said species (i), wherein the followings are determined in step (C):

   - the quantity of monomer (i) in the mixture M, which indicates the degree of conversion of the polymerization; and/or,
   - the mean hydrodynamic radius of the polymer (ii).

13. The method of anyone of claims 1 and 11, wherein the mixture M is diluted or dissolved in a medium identical to the carrier liquid used in the Taylor dispersion of step (A), before injecting it in the capillary.

14. The method of anyone of claims 1 to 13, wherein the internal surface of the capillary tube is non-covalently or covalently coated with a compound which limits or inhibits the interaction between the species (i) and (ii) and the inner surface of the capillary tube, said coated compound being chosen from the group consisting in PEO, cellulose derivatives, polyvinyl alcohol, polyacrylamide and its derivatives, polysiloxanes such as polydimethylsiloxane, anionic or cationic (mono- or double-chain) surfactants.

15. The method of anyone of claims 1 to 14, wherein the mixture M is introduced in the capillary tube together with a carrier liquid including a salt for example NaCl, or a buffer such as phosphate or borate.

16. The method of anyone of claims 1 to 15, wherein a plurality of samples, which each include a mixture of at least two species and having two different response coefficients on at least one detection device, are analysed according to steps (A) to (C) as defined in claim 1 in a same capillary tube, in a sequential way, the samples being injected successively in said capillary tube.

17. An analysis device comprising memorisation means, a processing means and an I/O interface to which is connected a detector capable of providing a Taylor signal, **characterized in that** the analysis device comprises means for the implementation of the analysis method according to anyone of the claims 1 to 16.

FIG.1

FIG.2 beginning

$$\sigma^2_{p,n}$$

$$H_n , D_n , R_{h,n}$$

$$A_{p,n} \; A_{m,n} \; \sigma^2_{p,n}$$

$$\Psi_n = \frac{A_{m,0} - A_{m,n}}{A_{m,0}}$$

$$x = \frac{1}{2}\left(\sqrt{\frac{4\beta_n - 3 - \alpha_n}{1-\alpha_n}} - 1\right)$$

$$\Psi_n = \frac{y_n}{y_n + \kappa(1-y_n)}$$

$$\Psi_n = \frac{1}{1-\kappa}\frac{A_0 - A_n}{A_0}$$

## FIG.2 end

Europäisches
Patentamt

European
Patent Office

Office européen
des brevets

# EUROPEAN SEARCH REPORT

Application Number

EP 08 30 5409

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COTTET H ET AL: "Taylor dispersion analysis of mixtures" ANALYTICAL CHEMISTRY, vol. 79, no. 23, 1 December 2007 (2007-12-01), pages 9066-9073, XP002512113 ISSN: 0003-2700 | 1,2,4,5, 9,13-17 | INV. G01N33/44 G01N15/02 ADD. G01N15/06 G01N30/00 |
| A | | 3,6-8, 10-12 | G01N35/08 |
| | * page 9070, left-hand column, paragraph 2 - page 9073, right-hand column, paragraph 1; figures * ----- | | |
| X | KELLY B ET AL: "Using Taylor dispersion profiles to characterize polymer molecular weight distributions" PCCP - PHYSICAL CHEMISTRY CHEMICAL PHYSICS, vol. 6, no. 24, 21 December 2004 (2004-12-21), pages 5523-5530, XP002512114 ISSN: 1463-9076 | 1,2,12, 13,16,17 | |
| A | * the whole document * ----- | 3-5 | TECHNICAL FIELDS SEARCHED (IPC) G01N |
| A | CALLENDAR R ET AL: "Diffusion coefficients for binary, ternary, and polydisperse solutions from peak-width analysis of Taylor dispersion profiles" JOURNAL OF SOLUTION CHEMISTRY, vol. 35, no. 3, March 2006 (2006-03), pages 353-379, XP002512115 ISSN: 0095-9782 * page 367, paragraph 4 - page 378, paragraph 1; figures 6-10; tables VI-X * ----- -/-- | 1,3-6, 9-12,17 | |

The present search report has been drawn up for all claims

| Place of search | Date of completion of the search | Examiner |
|---|---|---|
| Munich | 27 January 2009 | Johnson, Keith |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 08 30 5409

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | COTTET H ET AL: "Determination of dendrigraft poly-L-lysine diffusion coefficients by Taylor dispersion analysis" BIOMACROMOLECULES, vol. 8, no. 10, October 2007 (2007-10), pages 3235-3243, XP002512116 ISSN: 1525-7797 * the whole document * | 1,2,4, 10,12-17 | |
| A | WO 2008/031958 A (CENTRE NAT RECH SCIENT [FR]; COTTET HERVE [FR]; LE SAUX THOMAS [FR]) 20 March 2008 (2008-03-20) * abstract; claim 1; examples 1,2 * | 1,17 | |

TECHNICAL FIELDS
SEARCHED       (IPC)

The present search report has been drawn up for all claims

| Place of search | Date of completion of the search | Examiner |
|---|---|---|
| Munich | 27 January 2009 | Johnson, Keith |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 08 30 5409

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2009

| Patent document cited in search report | Publication date | Patent family member(s) | Publication date |
|---|---|---|---|
| WO 2008031958 A | 20-03-2008 | FR 2906032 A1 | 21-03-2008 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G. Taylor.** *Proc. Roy. Soc., A,* 1953, vol. 219, 186-203
- **R. Aris.** *Proc. Roy. Soc. Lond. A.,* 1956, vol. 235, 67-77
- **H. Cottet ; M. Martin ; A. Papillaud ; E. Souaïd ; H. Collet ; A. Commeyras.** *Biomacromolecules,* 2007, vol. 8, 3235-3243
- **A. J. S. Chapman ; D. M. Goodhall.** *Chromatography Today,* June 2008, vol. 1, 22-24
- *J. Phys. Chem.,* 1974, vol. 78, 2297-2301
- *Science,* 1994, vol. 266, 773-776